# EUROPEAN PATENT SPECIFICATION

(11) **EP 4 376 900 B1**
(45) Date of publication and mention of the grant of the patent: **30.04.2025**
(21) Application number: 22754037.4
(22) Date of filing: 18.07.2022
(51) Int. Cl.: A61L 9/20

(54) **A DISINFECTION LIGHTING DEVICE**
DESINFEKTIONSBELEUCHTUNGSVORRICHTUNG
DISPOSITIF D'ÉCLAIRAGE DE DÉSINFECTION

(30) Priority: 27.07.2021 EP 21187879
(43) Date of publication of application: 05.06.2024
(73) Proprietor: Signify Holding B.V., 5656 AE Eindhoven (NL)
(72) Inventor: VAN BOMMEL, Ties, 5656 AE Eindhoven (NL); HIKMET, Rifat, Ata, Mustafa, 5656 AE Eindhoven (NL)
(74) Representative: Verweij, Petronella Daniëlle
(86) International application number: PCT/EP2022/069984
(87) International publication number: WO 2023/006463

(56) References cited:
- WO-A1-2017/179082
- JP-A- 2006 185 672
- KR-A- 20200 030 728
- KR-B1- 102 219 256

## Description

### FIELD OF THE INVENTION

The present invention generally relates to the field of a lighting device, and in particular to a disinfection lighting device that is suitable for providing illumination as well as environmental disinfection functionality that is principally enabled by the photocatalysis process.

### BACKGROUND OF THE INVENTION

Light has the effect of disinfection. It is well known that violet and ultraviolet (UV) radiation emitted at certain wavelengths can destruct or neutralize microorganisms, such as fungi, viruses, and/or bacteria. It is also known that the photocatalysis process is activated by violet and ultraviolet (UV) radiation can be effectively used to inactivate microorganisms. In such a photocatalysis process, ionized chemical species can be generated for deactivating fungi, viruses, and/or bacteria. Besides, this photocatalysis process is well-known for the removal of organic pollutants in the gaseous phase such as volatile organic compounds (VOCs), having great potential applications to contaminant control in indoor environments such as residences, office buildings, factories, aircraft, and spacecraft.

Soma, R., et al., in U.S. Pat. No. 6,242,752 teaches the use of a photocatalytic film made of anatase-type Titanium Dioxide (TiO₂) on the lens of a lighting device such that, as the light originating from the lighting device shines through the TiO₂ film, the UV rays of the light activate the photocatalyst, causing it to break down the bacteria cell wall and resulting in the killing of the bacteria.

US2019234563A1 discloses a basic structure for a LED lamp, that is comprised of an aluminum core circuit board, and a base combination of violet-blue LEDs emitting visible electromagnetic radiation which wavelength has a peak that is comprised in given intervals. The envelope of the LED lamp enclosing the violet-blue LEDs has a photocatalytic material. Because of the technical and constructive characteristics of the lighting device, violet light emitted on the will allow it to break down the microbial load (fungi, viruses, and/or bacteria) present in any environment, without creating any adverse or dangerous effects for human beings or animals that live there while providing illumination.

JP2006185672A discloses an optical component for illumination, lamp device and luminaire.

### SUMMARY OF THE INVENTION

There is a desire to provide further improvement for the lighting device with disinfection functionality that is enabled by a photocatalysis process. In US2019234563A1, the violet light source is shown to be enclosed by an envelope and the material of the envelope is a photocatalytic material. The inventors recognize that the transmission of violet or UV light through such an arrangement can be limited in intensity or power which can adversely affect the efficiency of the photocatalysis process. Hence, this invention aims to provide a lighting device that emits visible electromagnetic radiation for the purpose of illumination and is also configured to provide improved environmental disinfection functionality by increasing the efficiency of the photocatalytic process.

These aims are accomplished by the invention, which is defined in claim 1.

According to a first aspect, this and other objects are achieved by a disinfection lighting device is provided. The disinfection lighting device comprises a first light source, a second light source, and an envelope. The first light source is adapted for, in operation, emitting visible light. The second light source is adapted for, in operation, emitting violet and/or ultraviolet (UV) light. The first light source is arranged inside the said envelope. The envelope comprises a recess and the second light source is arranged outside the said envelope and in the said recess. At least in the case that the second light source is adapted for, in operation, emitting violet light, the second light source is combined with a photocatalytic layer.

In other words, according to a first aspect of the invention, a disinfection lighting device is provided comprising a first light source, a second light source, and an envelope. The first light source is adapted for, in operation, emitting visible light. The second light source is adapted for, in operation, emitting violet and/or UV light. The envelope is adapted to enclose the first light source. The envelope further comprises a recess that extends into a volume that is enclosed by the envelope and the recess has a recess inner surface that is facing the volume and a recess outer surface that is opposite of the said recess inner surface. The second light source is arranged on the recess outer surface of the recess.

The envelope of the disinfection lighting device at least partly may cover the first light source. The second light source is located in the recess of the envelope, but out of the enclosure of the envelope. Therefore, the second light source may be exposed to an open environment, while the first light source is exposed to the enclosed environment within the envelope.

In the context of the present invention, the term 'recess' is to be understood as a section of the envelope that protrudes in an inward direction toward a volume of the enclosure by the said envelope.

In the context of the present invention, the term 'visible light' is to be understood as electromagnetic radiation in the range of wavelength that is visible to the human eye. Visible light is defined as light in a wavelength range from 420 to 780 nm.

In the context of the present invention, the term 'violet light' is to be understood as electromagnetic radiation in the range of wavelength from 380 to 420 nm.

In the context of the present invention, the term 'ultraviolet light' is to be understood as electromagnetic radiation in the range of wavelength from 100 to 380 nm.

The visible light emitted by the first light source may be white light having a color temperature between 2700 K and 6500 K. Alternatively, or in addition, the first light source may emit (also) colored light. The first light source may be configured to provide visible light which can be tuned in terms of color point and/or correlated color temperature. The white light may have a CRI of at least 80, preferably at least 85, more preferably at least 88, most preferably at least 90.

In the context of the present invention, the term 'violet light' is to be understood as electromagnetic radiation in the range of wavelength between 380 nanometers to 450 nanometers. Violet light is safer than ultraviolet light, but the disinfection performance of Violet light is lower than ultraviolet light. However, when violet light is used in combination with a photocatalytic layer, sufficient disinfection functionality may be realized.

In the context of the present invention, the term 'ultraviolet (UV) light' is to be understood as electromagnetic radiation in the range of wavelength between 100 nanometers to 380 nanometers. The second light source may be configured to emit either one or more types of UV light selected from the group: UV-A (315 to 380 nm), UV-B (280 to 315 nm), and UV-C (100 to 280 nm). The use of UV-A light is rather safe and can be used to inactivate bacteria, but UV-A is less efficient in inactivating viruses. The use of UV-B light is less safe compared to UV-A, but next to inactivating bacteria UV-B can be used to inactivate viruses although its effectiveness is lower than when using UV-C light. UV-C light can be used for inactivating both bacteria and viruses very efficiently, however, UV-C is less safe than UV-B light. An exception may be far UV-C light i.e. light in a wavelength range from 190 to 230 nm. Far UV-C light is very effective for killing both bacteria and viruses, while recent research shows its use may be rather safe because of the limited penetration depth of these wavelengths in the human skin and eyes.

UV light has been used for disinfection for over 100 years. Wavelengths between about 190 nm and 300 nm may be strongly absorbed by nucleic acids, which may result in defects in an organism's genome. This may be desired for inactivating (killing), bacteria and viruses, but may also have undesired side effects for humans. Therefore the selection of the wavelength of radiation, the intensity of radiation, and duration of irradiation may be limited in environments where people may reside such as offices, public transport, cinemas, restaurants, shops, etc., thus limiting the disinfection capacity. Especially in such environments, additional measures of disinfection may be advantageous to prevent the spread of bacteria and viruses such as influenza or novel (corona) viruses like COVID-19, SARS, and MERS.

It appears desirable to produce systems, that provide alternative ways for air treatment, such as disinfection. Further, existing systems for disinfection may not easily be implemented in existing infrastructure, such as in existing buildings like offices, hospitality areas, etc., and/or may not easily be able to serve larger spaces. This may again increase the risk of contamination. Further, incorporation in HVAC systems may not lead to desirable effects and appears to be relatively complex. Further, existing systems may not be efficient, or maybe relatively bulky, and may also not easily be incorporated in functional devices, such as e.g. luminaires.

Other disinfection systems may use one or more anti-microbial and/or antiviral means to disinfect a space or an object. Examples of such means may be chemical agents which may raise concerns. For instance, the chemical agents may also be harmful to people and pets.

In embodiments, the disinfecting light may especially comprise ultraviolet (UV) radiation (and/or optionally violet radiation), i.e., the light may comprise a wavelength selected from the ultraviolet wavelength range (and/or optionally the violet wavelength range). However, other wavelengths are herein not excluded. The ultraviolet wavelength range is defined as light in a wavelength range from 100 to 380 nm and can be divided into different types of UV light / UV wavelength ranges (Table 1). Different UV wavelengths of radiation may have different properties and thus may have different compatibility with human presence and may have different effects when used for disinfection (Table 1).

**Table 1: Properties of different types of UV, violet, and NIR wavelength light**

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Near Infrared (deep red) | NIR | 750-950 | + | +/- | +++ | | |
| Violet | V | 380-420 | +/- | - | + | | |
| Ultraviolet A | UV-A | 315-380 | + | - | + | | |
| Ultraviolet B | UV-B | 280-315 | + | +/- | +/- | + | |
| Near ultraviolet C | Near UV-C | 230-280 | ++ | ++ | - | | |
| Far ultraviolet C | Far UV-C | 190-230 | +++ | +++ | + | | +/- |
| Extreme ultraviolet C | Extreme UV-C | 100-190 | +++ | +++ | - | | + |

Each UV type/wavelength range may have different benefits and/or drawbacks. Relevant aspects may be (relative) sterilization effectiveness, safety (regarding radiation), and ozone production (as a result of its radiation). Depending on an application a specific type of UV light or a specific combination of UV light types may be selected and provides superior performance over other types of UV light. UV-A may be (relatively) safe and may inactivate (kill) bacteria, but maybe less effective in inactivating (killing) viruses. UV-B may be (relatively) safe when a low dose (i.e. low exposure time and/or low intensity) is used, may inactivate (kill) bacteria, and maybe moderately effective in inactivating (killing) viruses. UV-B may also have the additional benefit that it can be used effectively in the production of vitamin D in a skin of a person or animal. Near UV-C may be relatively unsafe, but may effectively inactivating, especially kill bacteria and viruses. Far UV may also be effective in inactivating (killing) bacteria and viruses but maybe (relatively to other UV-C wavelength ranges) (rather) safe. Far-UV light may generate some ozone which may be harmful to human beings and animals. Extreme UV-C may also be effective in inactivating (killing) bacteria and viruses but may be relatively unsafe. Extreme UV-C may generate ozone which may be undesired when exposed to human beings or animals. In some applications, ozone may be desired and may contribute to disinfection, but then it is shielding from humans and animals may be desired. Hence, the table "+" for ozone production especially implies that ozone is produced which may be useful for disinfection applications, but may be harmful to humans/animals when they are exposed to it. Hence, in many applications, this "+" may actually be undesired while in others, it may be desired. The types of light indicated in the above table may in embodiments be used to sanitize air and/or surfaces.

The terms "inactivating" and "killing" with respect to a virus may herein especially refer to damaging the virus in such a way that the virus can no longer infect and/or reproduce in a host cell, i.e., the virus may be (essentially) harmless after inactivation or killing.

Hence, in embodiments, the light may comprise a wavelength in the UV-A range. In further embodiments, the light may comprise a wavelength in the UV-B range. In further embodiments, the light may comprise a wavelength in the Near UV-C range. In further embodiments, the light may comprise a wavelength in the Far UV range. In further embodiments, the light may comprise a wavelength in the extreme UV-C range. The Near UV-C, the Far UV and the extreme UV-C ranges may herein also collectively be referred to as the UV-C range. Hence, in embodiments, the light may comprise a wavelength in the UV-C range. In other embodiments, the light may comprise violet radiation.

The second light source emitting violet and/or ultraviolet (UV) light in the exposed open environment may contribute towards air disinfection.

The envelope may be translucent or partially transparent toward the emitted visible light from the first light source.

The envelope may comprise surface features, textures, or optical components such as collimator, Fresnel lens segments, diffracting elements for realizing various form manipulation of the visible light or providing different visible lighting effects.

The envelope may be made from glass-type fragile materials.

The envelope may be made from translucent polymers such as PMMA, acrylic, or plexiglass.

The envelope may have multiple segments, each segment comprising a material that is different from other segments. For example, the envelope may be made from a glass-type material, while the recess part of the envelope is made from a polymer-type material. These two materials may be arranged to provide a single body of the envelope.

The envelope may comprise a diffuser configured to diffuse at least a portion of the visible light. The diffuser may have a reflectivity in the range from 30% to 80% for the visible light.

A diffuser with or within the envelope may allow a homogenized lighting effect.

The envelope may comprise an outer surface, and at least a portion of the outer surface may comprise a photocatalytic material.

Hence, the violet and/or ultraviolet (UV) light from the second light source can directly interact with the photocatalytic material for efficiently generating ionized chemical species that can inactivate and neutralize microorganisms. Therefore, this configuration may allow a disinfecting lighting device, where disinfection functionality by means of the photocatalysis process can be achieved relatively more efficiently.

The envelope may comprise an inner surface that is facing a volume that is enclosed by the envelope and an outer surface that is opposite of the said inner surface. Since the recess is being part of the envelope, the outer surface of the envelope is a continuous surface that meets the recess outer surface. And similarly, the inner surface of the envelope is a continuous surface that meets the recess inner surface. In other words, the recess inner surface or the recess outer surface is a portion of the inner or outer surface of the envelope, respectively.

At least a portion of the outer surface may comprise a photocatalytic material. In the context of the present invention, 'photocatalytic material' is understood to be as a material that can produce ionized chemical species from the chemical species that are in contact (physical or non-physical) with the material upon exposure to light, the light being visible, violet and/or UV light.

At least a portion of the outer surface of the envelope is arranged in the said recess.

At least a portion of the outer surface of the envelope is arranged in the said recess comprises a photocatalytic material.

At least a portion of the envelope comprises a photocatalytic material.

The envelope or at least a portion of the envelope may be made from a photocatalytic material.

The material of the envelope or at least a portion of the envelope may comprise a component that is a photocatalytic material.

A photocatalytic material may be embedded in at least a portion of the envelope.

At least a portion of the outer surface comprises a photocatalytic material, where the photocatalytic material is applied as a coating or layer on the portion of the outer surface of the envelope.

The photocatalytic material may be applied a coating or a layer on the at least a portion of the outer surface of the envelope that is arranged in the said recess.

Only the portion of the envelope that is located in the recess may comprise a photocatalytic material, wherein the rest of the envelope may comprise a material different from the photocatalytic material, or an inert material.

The disinfection lighting device may comprise an optical element that directs at least a portion of the violet and/or UV light emitted by the second light source impinging on the at least a portion of the outer surface comprising the photocatalytic material.

The disinfection lighting device may comprise an optical element that directs most of the violet and/or UV light emitted by the second light source that impinging on the at least a portion of the outer surface comprising the photocatalytic material.

An efficient photocatalysis process may be realized when violet and/or UV light is exposed to a photocatalytic material. Therefore, it may be desirable to have an optical element that directs violet and/or UV light emitted from the second light source towards the at least a portion of the outer surface comprising the photocatalytic material. The recess outer surface may represent this portion of the outer surface of the envelope. Especially when the recess outer surface comprises a photocatalytic material in the form of a layer or a coating.

The optical element may be attached or integrated with the second light source.

The optical element may be reflector type optics or refractive type optics that allow lateral distribution (parallel to an emission plane of the second light source) of the violet and/or UV light from the second light source.

At least a portion of the violet and/or UV light emitted by the second light source may impinge on the at least a portion of the outer surface being arranged in said recess.

A 20% to 80% of the violet and/or UV light emitted by the second light source may be impinging on the at least a portion of the outer surface of the envelope.

At least a portion of the violet and/or UV light emitted by the second light source may not be impinging on the portion of the outer surface of the envelope that is not part of the recess.

A 20% to 80% of the violet and/or UV light emitted by the second light source may not be impinging on the portion of the outer surface of the envelope that is not part of the recess.

A 20% to 80% of the violet and/or UV light emitted by the second light source may be impinging on the at least a portion of the outer surface that is arranged in the said recess.

A 20% to 80% of the violet and/or UV light emitted by the second light source may be impinging on the at least a portion of the outer surface that is arranged in the said recess having a photocatalytic material.

The photocatalytic material may comprise one of a Titanium Dioxide (TiO₂), Tungsten Trioxide (WO₃), Zinc Oxide (ZnO), Tin Oxide (SnO₂), Cerium Oxide (CeO₂), Silver Phosphate (Ag₃PO₄), and Cadmium Sulfide (CdS).

The photocatalytic material may comprise Titanium Dioxide (TiO₂), Tungsten Trioxide (WO₃), Zinc Oxide (ZnO), Tin Oxide (SnO₂), Cerium Oxide (CeO₂), Silver Phosphate (Ag₃PO₄), and/or Cadmium Sulfide (CdS), along with noble metal particles that may enable enhance photocatalysis. The noble metals can be Gold (Au), Silver (Ag), and/or Platinum (Pt). An alternative choice for this noble metal can be Copper (Cu).

The photocatalytic material may be applied as a thin-film, layer, or coating on the portion outer surface, potentially representing the recess outer surface with the following material, but not limited to these, Titanium Dioxide (TiO₂), Tungsten Trioxide (WO₃), Zinc Oxide (ZnO), Tin Oxide (SnO₂), Cerium Oxide (CeO₂), Silver Phosphate (Ag₃PO₄), and Cadmium Sulfide (CdS).

The recess may have a recess height (H1) that is 0.1 - 0.5 times to an envelope height (H2), and/or the recess may have a recess diameter (D1) that is 0.5 - 0.9 times an envelope diameter (D2).

The recess may need certain dimensions in terms of depth (described herein as height) within the envelope and length (described herein as diameter) along with the envelope. The recess height (H1) may be 0.1 - 0.5 times to an envelope height (H2) to facilitate the second light source. In addition, the recess diameter (D1) maybe 0.5 - 0.9 times an envelope diameter (D2) to have sufficient surface area comprising photocatalytic material for realizing sufficient disinfection effect.

The value of the recess diameter (D1) or the recess length may be the maximum measurable value.

The value of the envelope diameter (D2) or envelope length may be the maximum measurable value.

The value of the recess height (H1) may be the maximum measurable value.

The value of the envelope height (H2) may be the maximum measurable value.

The recess may comprise a recess portion having a circular or a polygonal circumference.

The recess may comprise a recess portion having an elliptical circumference.

The disinfection lighting device may comprise a fan that is configured to provide a forced airflow to the recess.

The disinfection lighting device may comprise a fan between an air inlet and air outlet, and the air outlet is configured to a forced airflow to the recess. The airflow to the recess, where the recess outer surface that is part of the outer surface of the envelope having a photocatalytic material may sustainably produce ionized chemical species to neutralize and inactivating microorganisms.

The fan may be located within a housing of the disinfection lighting device. The air inlet(s) may be located around the housing of the disinfection lighting device for collecting sufficient airflow towards the air outlet.

The second light source may be configured to provide violet light having a dominant peak in the wavelength range from 380 to 420, preferably in a wavelength range from 390 to 415 nm, more preferably in a wavelength range from 400 to 410 nm, most preferably in a wavelength range from 403 to 407 nm.

Photocatalytic material such as Titanium Dioxide (TiO₂) in the anatase phase may offer very efficient photocatalysis when exposed to violet light having a dominant peak in the wavelength range between 400 to 410 nm.

The disinfection lighting device may comprise a controller for individually controlling the visible light emitted by the first light source and the violet and/or UV light emitted by the second light source.

The controller may be located within a housing of the disinfection lighting device.

The controller may allow individual control of the first and the second lighting sources.

The controller may allow combined control of the first and the second lighting sources. For example, when the second light source is emitting the violet and/or UV light for activating disinfection functionality, the first light source may provide specific color or signal based on visible light to inform active disinfection functionality modus.

The controller may allow at least one of color change, turning ON/OFF, and dimming of the first light source.

The controller may allow at least one of turning ON/OFF and intensity control of the second light source.

According to a second aspect of the present invention, a lamp is provided that comprises the disinfection lighting device.

Alternatively, according to a second aspect of the present invention, a lamp is provided that comprises the disinfection lighting device. The disinfection lighting device comprises the envelope that comprises a diffuser configured to diffuse at least a portion of the visible light, and the envelope comprises an outer surface. At least a portion of the outer surface comprises a photocatalytic material, the at least a portion of the outer surface of the envelope is arranged in the said recess; and at least a portion of the violet and/or UV light emitted by the second light source impinges on the at least a portion of the outer surface being arranged in said recess.

The lamp is provided that comprises the disinfection lighting device, where the lamp further comprises a base for securing the lamp in a fitting by inserting the base (in a socket of the fitting. The base may have a base electrode arrangement and the light source may be electrically connected to the base electrode arrangement.

The fitting may comprise a socket for securing the lamp in the fitting by receiving a base of the lamp through an opening of the socket, wherein the socket has a socket electrode arrangement for making an electrical connection to a base electrode arrangement of the lamp.

The fitting and the lamp together may be combined together to provide a luminaire that is adapted to provide illumination and disinfection.

It is noted that the invention relates to all possible combinations of features recited in the claims. Other objectives, features, and advantages of the present inventive concept will appear from the following detailed disclosure, from the attached claims as well as from the drawings. A feature described in relation to one of the aspects may also be incorporated in the other aspect, and the advantage of the feature is applicable to all aspects in which it is incorporated.

### BRIEF DESCRIPTION OF THE DRAWINGS

The above, as well as additional objects, features, and advantages of the disclosed devices, methods, and systems, will be better understood through the following illustrative and non-limiting detailed description of embodiments of devices, methods, and systems, with reference to the appended drawings, in which:
Figs. 1(a) and (b) show a cross-sectional view and a top view of a lamp, respectively;
Fig. 2 shows a cross-sectional view and a top view of a lamp; and
Fig. 3 shows a cross-sectional view and a top view of a lamp having an alternative configuration.

As illustrated in the figures, the sizes of layers and regions are exaggerated for illustrative purposes and, thus, are provided to illustrate the general structures of embodiments of the present invention. Like reference numerals refer to like elements throughout.

### DETAILED DESCRIPTION

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

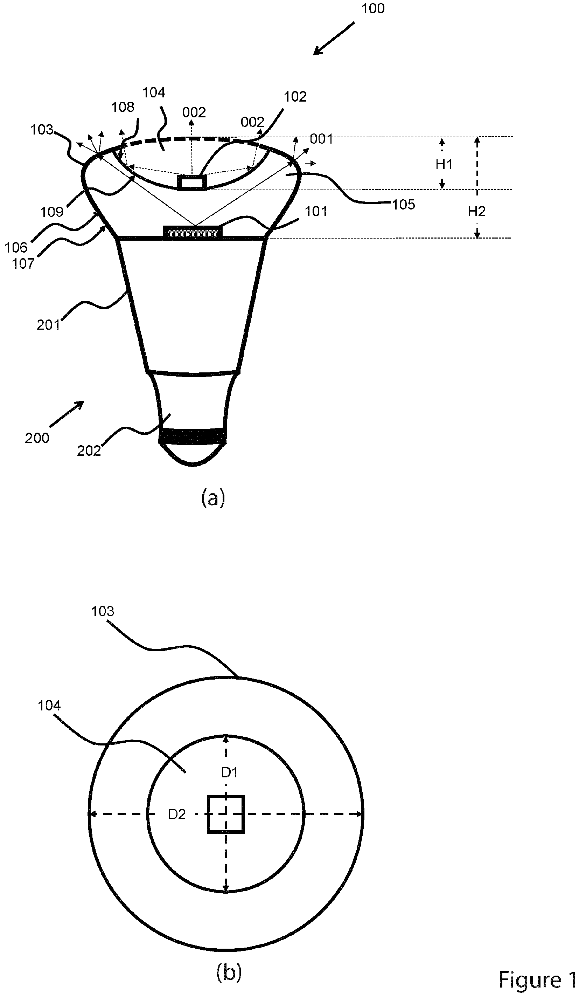

Referring initially to Figure 1(a), a cross-sectional view of a lamp 200 comprising a disinfection lighting device 100 is shown. The lamp 200 comprises a housing 201 and a base 202. The base 202 as shown in the figure is an Edison base. However, a pin-type base or another form of the base can be used as well. The base 202 is for securing the lamp 200 in a fitting. The fitting may comprise a socket for securing the lamp 200 in the fitting by receiving a base 202 of the lamp through an opening of the socket, wherein the socket has a socket electrode arrangement for making an electrical connection to a base electrode arrangement (not shown in the figure) of the lamp 200. The housing 201 may cover the base electrode that is in electrical contact with the first light source 101 and the second light source 102. Also, the housing 201 may serve as the heat sinks for electronics as well as the first light source 101 and the second light source 102.

The disinfection lighting device 100 comprises the first light source 101, the second light source 102, and an envelope 103. The first light source 101 is arranged inside the envelope 103. Therefore, the envelope 103 is adapted to enclose the first light source 101. The envelope 103 comprises a recess 104 and the second light source is arranged in the recess 104, but outside of the envelope 103. The recess 104 extends into a volume 105 that is enclosed by the envelope 103. The envelope 103 has an inner surface 106 and opposite to that an outer surface 107. The inner surface 106 is facing the volume 105. A portion of the outer surface 108 is shown to be the recess outer surface 108, which is a part of the outer surface 107 of the envelope 103. Similarly, a portion of the inner surface 109 is shown to be the recess inner surface 109 that is part of the inner surface 106 of the envelope 103. Because the recess 104 is part of the envelope 103, the inner surface 106 of the envelope 103 is a continuation with the recess inner surface 109, and the outer surface 107 of the envelope 103 is a continuation with the recess outer surface 108. The second light source 102 is arranged on the recess outer surface 108.

In operation, the first light source 101 is configured to emit visible light 001, and the second light source 102 is configured to emit violet and/or ultraviolet (UV) light 002. As shown in Figure 1(a), the visible 001 emitted by the first light source 101 is transmitted through the envelope 103. Therefore, the envelope 103 is at least partially transparent to the visible light 001. The visible light 001 may also at least partially transmit through the recess 104. On the other hand, a portion of the violet and/or ultraviolet (UV) light 002 emitted from the second light source 102 is free to propagate towards an open space in front of envelope 103. And other portion of the violet and/or ultraviolet (UV) light 002 emitted from the second light source 102 is impinging on the portion of the outer surface 108 of the envelope 103 and subsequently reflected towards the open space in front of envelope 103, dictated by the refractive property of the said that is the recess outer surface 108. At least a portion of the violet and/or ultraviolet (UV) light 002 may go into the volume 105 enclosed by the envelope 103 if the envelope 103 is at least partially transmissive to the violet and/or ultraviolet (UV) light 002.

In Figure 1(a), the recess 104 is shown to have a curved shape. One may define it to be a spherical cap or a spherical segment depending on the preference.

The envelope 103 may comprise a photocatalytic material, such as Titanium Dioxide (TiO₂), Tungsten Trioxide (WO₃), Zinc Oxide (ZnO), Tin Oxide (SnO₂), Cerium Oxide (CeO₂), Silver Phosphate (Ag₃PO₄), and/or Cadmium Sulfide (CdS).

Or, at least a portion of the outer surface 108, herein referred to as the recess outer surface 108 may comprise a photocatalytic material such as Titanium Dioxide (TiO₂), Tungsten Trioxide (WO₃), Zinc Oxide (ZnO), Tin Oxide (SnO₂), Cerium Oxide (CeO₂), Silver Phosphate (Ag₃PO₄), and/or Cadmium Sulfide (CdS).

The envelope 103 or at least a portion of the envelope 103, for example, the recess 104 may be made from the photocatalytic material.

Hence, the violet and/or ultraviolet (UV) light 002 from the second light source 102 can directly interact with the photocatalytic material for efficiently generating ionized chemical species that can inactivate and neutralize microorganisms. Therefore, this configuration that is shown in Figure 1(a) may allow a disinfecting lighting device 100, where disinfection functionality by means of the photocatalysis process can be achieved relatively more efficiently.

The photocatalytic material can be at least partially transparent to the visible light 001. For example, the envelope 103 may be diffusive towards the visible light 001 for providing a more homogenized lighting effect.

Alternatively, the photocatalytic material or the photocatalytic material applied as a layer or coating may be at least partially reflective towards the violet and/or ultraviolet (UV) light 002, but at least partially transmissive towards the visible light 001.

In Figure 1(a), the envelope 103 is shown to have an envelope height (H2) and the recess 104 is shown to have a maximum recess height (H1). H1 is approximately 0.5 times the H2. The recess height (H1) maybe 0.1 - 0.5 times to an envelope height (H2) to accommodate the second light source in recess.

In Figure 1(b), the top view of the disinfection lighting device 100 is shown. The recess 104 has a spherical cap (part of a sphere) shape. Hence, the circumference of the recess 104 is shown to be a circle. Similarly, the circumference of the envelope 103 is shown to be a circle. The recess diameter (D1) maybe 0.5 - 0.9 times of an envelope diameter (D2) to have sufficient surface area comprising photocatalytic material for realizing sufficient disinfection effect. The circumference of the recess 104 may be alternatively a polygon or an ellipse.

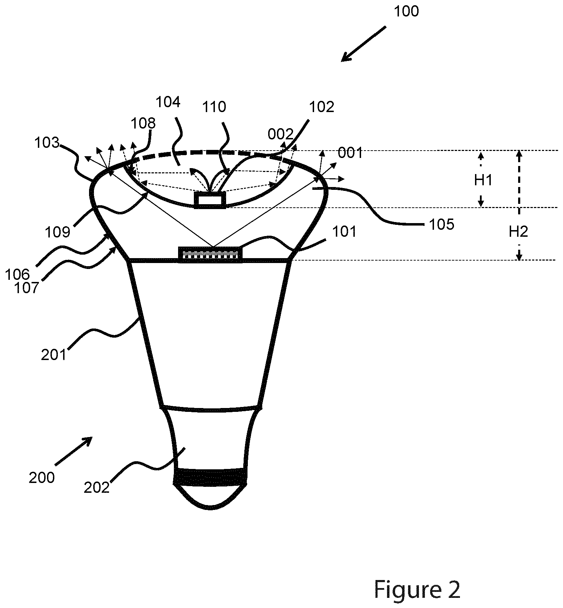

Figure 2 shows a cross-sectional view of the disinfection lighting device 100 similar to that shown in Figure 1(a), but additional comprising an optical element 110. As shown in Figure 2, the optical element 110 is depicted to be curved reflector that ensures that substantial violet and/or UV light 002 from the second light source 102 is directed towards the recess 104. It may be desirable to have an optical element that directs violet and/or UV light 002 emitted from the second light source 102 towards the at least a portion of the outer surface 108 comprising the photocatalytic material. The recess outer surface 108 may represent this portion of the outer surface 108 of the envelope 107.

The optical element 110 may be attached or integrated with the second light source 102.

The optical element 110 may be a reflector type optics (as shown in Figure 2) or refractive type optics that allow lateral distribution (parallel to an emission plane of the second light source) of the violet and/or UV light 002 from the second light source 102 onto the at least a portion of the outer surface 108 of the envelope 103 comprising the photocatalytic material.

The optical element 110 may be configured such that a 20% to 80% of the violet and/or UV light emitted 002 by the second light source 102 may be impinging on the at least a portion of the outer surface 108 of the envelope 103 comprising the photocatalytic material. The optical element 110 may be configured such that no violet and/or UV light 002 emitted by the second light 102 to the part of the outer surface 107 that does not have the photocatalytic material.

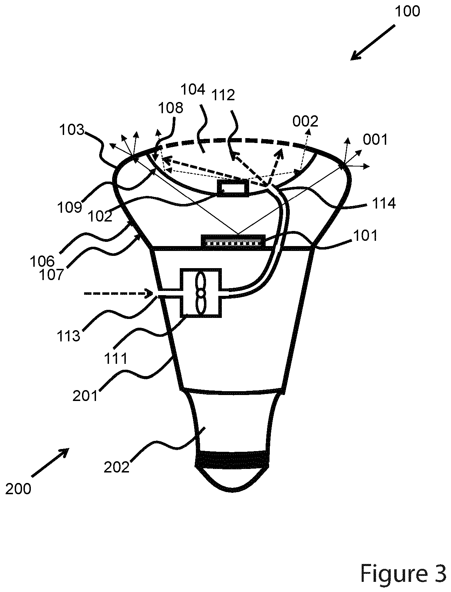

Figure 3 shows a cross-sectional view of a lamp 200 comprising a disinfection lighting device 100 as shown in Figures 1(a) and 2. Similar to these earlier figures, the reference numerals are the same and represent the same components. However, the disinfection lighting device 100 is shown to further comprise a fan 111 that is configured to provide a forced airflow 112 to the recess 104. The fan 111 is located in the housing 201. The fan 111 has an access to an air inlet 113 that draws in air that is expelled out from an air outlet 114. The air outlet 114 is shown to be facing the recess 104 and located on the recess outer surface 108. This forced air flow 112 to the recess 104, where the recess outer surface 108 that is part of the outer surface 107 of the envelope 103 having a photocatalytic material may sustainably produce ionized chemical species to neutralize and inactivating microorganisms. The air inlet(s) 113 may be located around the housing 201 of the disinfection lighting device 100 for collecting sufficient airflow towards the air outlet(s) 114. The disinfection lighting device 100 may comprise a plurality of air outlets that are arranged around the recess 104.

## Claims

1. A disinfection lighting device (100) comprising:
- a first light source (101) adapted for, in operation, emitting visible light (001);
- a second light source (102) adapted for, in operation, emitting violet and/or ultraviolet (UV) light (002); and
- an envelope (103);
**characterized in that** the first light source (101) is arranged inside the said envelope (103);
wherein the envelope (103) comprises a recess (104); and
wherein the second light source (102) is arranged outside the said envelope (103) and in the said recess (104), and
wherein at least when the second light source (102) is adapted for, in operation, emitting violet light, the second light source (102) is combined with a photocatalytic layer.

2. The disinfection lighting device (100) according to claim 1, wherein the envelope (103) comprises a diffuser configured to diffuse at least a portion of the visible light (001).

3. The disinfection lighting device (100) according to any one of the preceding claims, wherein the envelope (103) comprises an outer surface (107), and wherein at least a portion of the outer surface (108) comprises a photocatalytic material.

4. The disinfection lighting device (100) according to claim 3, wherein the at least a portion of the outer surface (108) of the envelope (130) is arranged in the said recess (104).

5. The disinfection lighting device (100) according to any one of claims from 3 to 4, wherein the disinfection lighting device (100) comprises an optical element (110) that directs at least a portion of the violet and/or UV light (002) emitted by the second light source (102) imping on the at least a portion of the outer surface (108) comprising the photocatalytic material.

6. The disinfection lighting device (100) according to claim 5, wherein at least a portion of the violet and/or UV light (002) emitted by the second light source (102) impinges on the at least a portion of the outer surface (108) being arranged in said recess (104).

7. The disinfection lighting device (100) according to any one of the preceding claims, wherein 20% to 80% of the violet and/or UV light (002) emitted by the second light source (102) is impinging on the at least a portion of the outer surface (108) of the envelope (103).

8. The disinfection lighting device (100) according to any one of the preceding claims, wherein 20% to 80% of the violet and/or UV light (002) emitted by the second light source (102) is impinging on the at least a portion of the outer surface (108) that is arranged in the said recess (104).

9. The disinfection lighting device (100) according to any one of claims from 3 to 8, wherein the photocatalytic material comprises one of a Titanium Dioxide, Tungsten Trioxide, Zinc Oxide, Tin Oxide, Cerium Oxide, Silver Phosphate, and Cadmium Sulfide.

10. The disinfection lighting device (100) according to any one of the preceding claims, wherein the recess (104) has a recess height (H1) being 0.1 - 0.5 times an envelope height (H2), and/or wherein the recess (104) has a recess diameter (D1) being 0.5 - 0.9 times an envelope diameter (D2).

11. The disinfection lighting device (100) according to any one of the preceding claims, wherein the recess (104) comprises a recess portion having a circular or a polygonal circumference.

12. The disinfection lighting device (100) according to any one of the preceding claims, wherein the disinfection lighting device (100) comprises a fan (111) that is configured to provide a forced airflow (112) to the recess (104).

13. The disinfection lighting device (100) according to any one of the preceding claims, wherein the second light source (102) is configured to provide violet light having a dominant peak in the wavelength range between 400 to 410 nm.

14. The disinfection lighting device (100) according to any one of the preceding claims, wherein the disinfection lighting device (100) comprises a controller for individually controlling the visible light (001) emitted by the first light source (101) and the violet and/or UV light (002) emitted by the second light source (102).

15. A lamp (200) comprising the disinfection lighting device (100) according to any one of the preceding claims,
- wherein the envelope (103) comprises a diffuser configured to diffuse at least a portion of the visible light (001);
- wherein the envelope (103) comprises an outer surface (107), and wherein at least a portion of the outer surface (108) comprises a photocatalytic material;
- wherein the at least a portion of the outer surface (108) of the envelope (130) is arranged in the said recess (104); and
- wherein at least a portion of the violet and/or UV light (002) emitted by the second light source (102) impinges on the at least a portion of the outer surface (108) being arranged in said recess (104).

## Patentansprüche

1. Desinfektionsbeleuchtungsvorrichtung (100), umfassend:
- eine erste Lichtquelle (101), die angepasst ist, um im Betrieb sichtbares Licht (001) zu emittieren;
- eine zweite Lichtquelle (102), die angepasst ist, um im Betrieb violettes und/oder ultraviolettes Licht (UV-Licht) (002) zu emittieren; und
- eine Kolben (103);
**dadurch gekennzeichnet, dass** die erste Lichtquelle (101) innerhalb des Kolbens (103) angeordnet ist;
wobei der Kolben (103) eine Aussparung (104) umfasst; und
wobei die zweite Lichtquelle (102) außerhalb des Kolbens (103) und in der Vertiefung (104) angeordnet ist, und
wobei zumindest dann, wenn die zweite Lichtquelle (102) dazu ausgebildet ist, im Betrieb violettes Licht zu emittieren, die zweite Lichtquelle (102) mit einer photokatalytischen Schicht kombiniert ist.

2. Desinfektionsbeleuchtungsvorrichtung (100) nach Anspruch 1, wobei der Kolben (103) einen Diffusor umfasst, der konfiguriert ist, um mindestens einen Teil des sichtbaren Lichts (001) zu streuen.

3. Desinfektionsbeleuchtungsvorrichtung (100) nach einem der vorhergehenden Ansprüche, wobei der Kolben (103) eine Außenoberfläche (107) umfasst, und wobei mindestens ein Abschnitt der Außenoberfläche (108) ein photokatalytisches Material umfasst.

4. Desinfektionsbeleuchtungsvorrichtung (100) nach Anspruch 3, wobei mindestens ein Abschnitt der Außenoberfläche (108) des Kolbens (130) in der Vertiefung (104) angeordnet ist.

5. Desinfektionsbeleuchtungsvorrichtung (100) nach einem der Ansprüche 3 bis 4, wobei die Desinfektionsbeleuchtungsvorrichtung (100) ein optisches Element (110) umfasst, das mindestens einen Abschnitt des von der zweiten Lichtquelle (102) emittierten violetten und/oder UV-Lichts (002) auf mindestens einen Abschnitt der Außenoberfläche (108) lenkt, der das photokatalytische Material umfasst.

6. Desinfektionsbeleuchtungsvorrichtung (100) nach Anspruch 5, wobei mindestens ein Abschnitt des von der zweiten Lichtquelle (102) emittierten violetten und/oder UV-Lichts (002) auf mindestens einem Abschnitt der Außenoberfläche (108) auftrifft, der in der Vertiefung (104) angeordnet ist.

7. Desinfektionsbeleuchtungsvorrichtung (100) nach einem der vorhergehenden Ansprüche, wobei 20 % bis 80 % des von der zweiten Lichtquelle (102) emittierten violetten und/oder UV-Lichts (002) auf mindestens einen Abschnitt der Außenoberfläche (108) des Kolbens (103) auftreffen.

8. Desinfektionsbeleuchtungsvorrichtung (100) nach einem der vorhergehenden Ansprüche, wobei 20 % bis 80 % des von der zweiten Lichtquelle (102) emittierten violetten und/oder UV-Lichts (002) auf mindestens einen Abschnitt der Außenoberfläche (108) auftreffen, der in der Vertiefung (104) angeordnet ist.

9. Desinfektionsbeleuchtungsvorrichtung (100) nach einem der Ansprüche 3 bis 8, wobei das photokatalytische Material eines umfasst von einem Titandioxid, Wolframtrioxid, Zinkoxid, Zinnoxid, Ceroxid, Silberphosphat und Cadmiumsulfid.

10. Desinfektionsbeleuchtungsvorrichtung (100) nach einem der vorhergehenden Ansprüche, wobei die Aussparung (104) eine Aussparungshöhe (H1) aufweist, die das 0,1-0,5-fache einer Kolbenhöhe (H2) beträgt, und/oder wobei die Aussparung (104) einen Aussparungsdurchmesser (D1) aufweist, der das 0,5-0,9-fache eines Kolbendurchmessers (D2) beträgt.

11. Desinfektionsbeleuchtungsvorrichtung (100) nach einem der vorhergehenden Ansprüche, wobei die Aussparung (104) einen Aussparungsabschnitt mit einem kreisförmigen oder polygonalen Umfang umfasst.

12. Desinfektionsbeleuchtungsvorrichtung (100) nach einem der vorhergehenden Ansprüche, wobei die Desinfektionsbeleuchtungsvorrichtung (100) einen Ventilator (111) umfasst, der konfiguriert ist, um einen erzwungenen Luftstrom (112) zu der Vertiefung (104) bereitzustellen.

13. Desinfektionsbeleuchtungsvorrichtung (100) nach einem der vorhergehenden Ansprüche, wobei die zweite Lichtquelle (102) konfiguriert ist, um violettes Licht mit einem dominanten Peak im Wellenlängenbereich zwischen 400 und 410 nm bereitzustellen.

14. Desinfektionsbeleuchtungsvorrichtung (100) nach einem der vorhergehenden Ansprüche, wobei die Desinfektionsbeleuchtungsvorrichtung (100) eine Steuerung zum individuellen Steuern des von der ersten Lichtquelle (101) emittierten sichtbaren Lichts (001) und des von der zweiten Lichtquelle (102) emittierten violetten und/oder UV-Lichts (002) umfasst.

15. Leuchtmittel (200), umfassend die Desinfektionsbeleuchtungsvorrichtung (100) nach einem der vorstehenden Ansprüche,
- wobei der Kolben (103) einen Diffusor umfasst, der konfiguriert ist, um mindestens einen Abschnitt des sichtbaren Lichts (001) zu streuen;
- wobei der Kolben (103) eine Außenoberfläche (107) umfasst, und wobei mindestens ein Abschnitt der Außenoberfläche (108) ein photokatalytisches Material umfasst;
- wobei mindestens ein Abschnitt der Außenoberfläche (108) des Kolbens (130) in der Vertiefung (104) angeordnet ist; und
- wobei mindestens ein Abschnitt des von der zweiten Lichtquelle (102) emittierten violetten und/oder UV-Lichts (002) auf mindestens einen Abschnitt der Außenoberfläche (108) auftrifft, der in der Vertiefung (104) angeordnet ist.

## Revendications

1. Dispositif d'éclairage pour la désinfection (100) comprenant :
- une première source de lumière (101) adaptée à émettre, en fonctionnement, de la lumière visible (001) ;
- une seconde source de lumière (102) adaptée à émettre, en fonctionnement, de la lumière violette et/ou ultraviolette (UV) (002) ; et
- une enveloppe (103) ;
**caractérisé en ce que** la première source de lumière (101) est agencée à l'intérieur de ladite enveloppe (103) ;
dans lequel l'enveloppe (103) comprend une évidement (104) ; et
dans lequel la seconde source de lumière (102) est agencée à l'extérieur de ladite enveloppe (103) et dans ledit évidement (104), et
dans lequel, au moins lorsque la seconde source de lumière (102) est adaptée à émettre, en fonctionnement, de la lumière violette, la seconde source de lumière (102) est combinée à une couche photocatalytique.

2. Dispositif d'éclairage pour la désinfection (100) selon la revendication 1, dans lequel l'enveloppe (103) comprend un diffuseur configuré pour diffuser au moins une partie de la lumière visible (001).

3. Dispositif d'éclairage pour la désinfection (100) selon l'une quelconque des revendications précédentes, dans lequel l'enveloppe (103) comprend une surface extérieure (107), et dans lequel au moins une partie de la surface extérieure (108) comprend un matériau photocatalytique.

4. Dispositif d'éclairage pour la désinfection (100) selon la revendication 3, dans lequel l'au moins une partie de la surface extérieure (108) de l'enveloppe (130) est agencée dans ledit évidement (104).

5. Dispositif d'éclairage pour la désinfection (100) selon l'une quelconque des revendications 3 à 4, dans lequel le dispositif d'éclairage pour la désinfection (100) comprend un élément optique (110) qui dirige au moins une partie de la lumière violette et/ou **UV** (002) émise par la seconde source de lumière (102) heurtant l'au moins une partie de la surface extérieure (108) comprenant le matériau photocatalytique.

6. Dispositif d'éclairage pour la désinfection (100) selon la revendication 5, dans lequel au moins une partie de la lumière violette et/ou **UV** (002) émise par la seconde source de lumière (102) heurte l'au moins une partie de la surface extérieure (108) agencée dans ledit évidement (104).

7. Dispositif d'éclairage pour la désinfection (100) selon l'une quelconque des revendications précédentes, dans lequel 20 % à 80 % de la lumière violette et/ou UV (002) émise par la seconde source de lumière (102) heurte l'au moins une partie de la surface extérieure (108) de l'enveloppe (103).

8. Dispositif d'éclairage pour la désinfection (100) selon l'une quelconque des revendications précédentes, dans lequel 20 % à 80 % de la lumière violette et/ou UV (002) émise par la seconde source de lumière (102) heurte l'au moins une partie de la surface extérieure (108) qui est agencée dans ledit évidement (104).

9. Dispositif d'éclairage pour la désinfection (100) selon l'une quelconque des revendications 3 à 8, dans lequel le matériau photocatalytique comprend l'un parmi du dioxyde de titane, du trioxyde de tungstène, de l'oxyde de zinc, de l'oxyde d'étain, de l'oxyde de cérium, du phosphate d'argent et du sulfure de cadmium.

10. Dispositif d'éclairage pour la désinfection (100) selon l'une quelconque des revendications précédentes, dans lequel l'évidement (104) a une hauteur d'évidement (H1) représentant 0,1 à 0,5 fois une hauteur d'enveloppe (H2), et/ou dans lequel l'évidement (104) a un diamètre d'évidement (D1) représentant 0,5 à 0,9 fois un diamètre d'enveloppe (D2).

11. Dispositif d'éclairage pour la désinfection (100) selon l'une quelconque des revendications précédentes, dans lequel l'évidement (104) comprend une partie d'évidement ayant une circonférence circulaire ou polygonale.

12. Dispositif d'éclairage pour la désinfection (100) selon l'une quelconque des revendications précédentes, dans lequel le dispositif d'éclairage pour la désinfection (100) comprend un ventilateur (111) qui est configuré pour fournir un flux d'air forcé (112) à l'évidement (104).

13. Dispositif d'éclairage pour la désinfection (100) selon l'une quelconque des revendications précédentes, dans lequel la seconde source de lumière (102) est configurée pour fournir une lumière violette ayant un pic dominant dans la plage de longueurs d'onde comprise entre 400 et 410 nm.

14. Dispositif d'éclairage pour la désinfection (100) selon l'une quelconque des revendications précédentes, dans lequel le dispositif d'éclairage pour la désinfection (100) comprend un dispositif de commande pour commander individuellement la lumière visible (001) émise par la première source de lumière (101) et la lumière violette et/ou UV (002) émise par la seconde source de lumière (102).

15. Lampe (200) comprenant le dispositif d'éclairage pour la désinfection (100) selon l'une quelconque des revendications précédentes.
- dans laquelle l'enveloppe (103) comprend un diffuseur configuré pour diffuser au moins une partie de la lumière visible (001) ;
- dans laquelle l'enveloppe (103) comprend une surface extérieure (107), et dans laquelle au moins une partie de la surface extérieure (108) comprend un matériau photocatalytique ;
- dans laquelle l'au moins une partie de la surface extérieure (108) de l'enveloppe (130) est agencée dans ledit évidement (104) ; et
- dans laquelle au moins une partie de la lumière violette et/ou UV (002) émise par la seconde source de lumière (102) heurte l'au moins une partie de la surface extérieure (108) agencée dans ledit évidement (104).
